(19) Europäisches Patentamt

European Patent Office

Office européen des brevets

(11) **EP 0 615 425 B1**

(12) **FASCICULE DE BREVET EUROPEEN**

(45) Date de publication et mention
de la délivrance du brevet:
**14.08.1996 Bulletin 1996/33**

(21) Numéro de dépôt: **93901767.9**

(22) Date de dépôt: **25.11.1992**

(51) Int. Cl.$^6$: **A61B 5/113**

(86) Numéro de dépôt international:
**PCT/FR92/01092**

(87) Numéro de publication internationale:
**WO 93/10707 (10.06.1993 Gazette 1993/14)**

(54) **PROCEDE ET DISPOSITIF DE REEDUCATION RESPIRATOIRE**

VERFAHREN UND GERäT ZUR ATMUNGSUMSCHULUNG

BREATHING REEDUCATION DEVICE AND METHOD

(84) Etats contractants désignés:
**BE DE FR GB IT NL**

(30) Priorité: **28.11.1991 FR 9115313**

(43) Date de publication de la demande:
**21.09.1994 Bulletin 1994/38**

(73) Titulaire: **UNIVERSITE JOSEPH FOURIER**
**F-38041 Grenoble Cédex (FR)**

(72) Inventeurs:
• **BACONNIER, Pierre**
**F-3800 Grenoble (FR)**

• **BENCHETRIT, Gila**
**F-38000 Grenoble (FR)**
• **ESTEVE, François**
**F-38190 Crolles (FR)**
• **BLANC-GRAS, Nathalie**
**F-38610 Gières (FR)**

(74) Mandataire: **de Beaumont, Michel**
**1bis, rue Champollion**
**38000 Grenoble (FR)**

(56) Documents cités:
**WO-A-86/00793        US-A- 4 258 718**
**US-A- 4 373 534**

## Description

La présente invention concerne le domaine de la rééducation des mouvements ventilatoires chez des patients pour lesquels ont été prescrites des séances de kinésithérapie respiratoire.

Des séances de kinésithérapie respiratoire peuvent être prescrites pour des personnes qui présentent un état général pathologique que l'on attribue à une insuffisance respiratoire. Cette insuffisance respiratoire se traduit par exemple, par un essouflement rapide au moindre effort physique. On peut également prescrire ces séances à des personnes ayant subi des interventions affectant la fonction respiratoire, telles qu'une ablation de poumon, pour qu'elles s'habituent à respirer dans leur nouvel état.

Actuellement, les rééducations respiratoires sont pratiquées en présence d'un kinésithérapeute à raison de plusieurs séances hebdomadaires. Celui-ci corrige les mouvements ventilatoires du patient dans le but d'améliorer l'efficacité de sa ventilation. Les corrections indiquées par le kinésithérapeute sont guidées par ses connaissances de l'efficacité ventilatoire spécifique à chaque type de mouvement (respiration abdominale, thoracique...).

Les kinésithérapeutes disposent d'un certain nombre d'appareils qui permettent une meilleur suivi des mouvements indiqués, mais qui ne sont pas destinés à mesurer la ventilation. Il existe des appareils qui pourraient être utilisés pour mesurer la ventilation pendant une séance de rééducation, mais leur manipulation est trop peu pratique pour une utilisation courante dans un cabinet de kinésithérapeute.

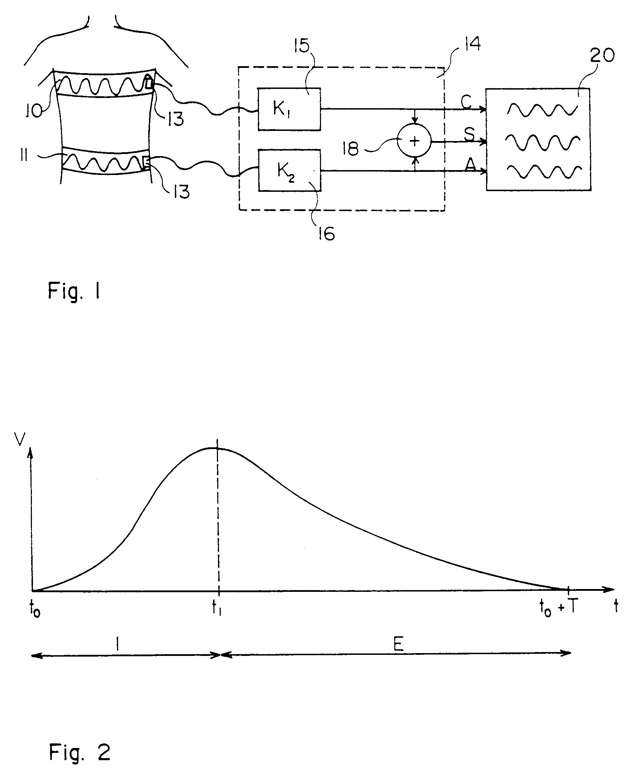

La figure 1 illustre un tel appareil. Il s'agit d'un pléthysmographe à spires inductives tel que celui décrit dans le brevet anglais 1596298.

L'appareil est composé d'une bande comprenant une spire thoracique 10 et d'une bande comprenant une spire abdominale 11, disposées respectivement autour de la poitrine et du ventre d'un patient. Chacune des spires est à section variable et détermine la fréquence d'un oscillateur 13 associé solidaire de la bande. Les spires sont à section variable grâce, par exemple, comme cela est représenté, à une disposition en sinusoïde dans la largeur des bandes. Les oscillateurs 13 sont reliés à un circuit de traitement 14 comprenant un convertisseur fréquence-tension par oscillateur, respectivement 15 de gain K1 et 16 de gain K2. Le convertisseur 15 fournit une tension C représentative de la section du thorax et le convertisseur 16 fournit une tension A représentative de la section de l'abdomen. Les tensions C, A et leur somme S fournie par un sommateur 18 peuvent être visualisées par tout dispositif 20 de mesure de tension, tel qu'un voltmètre, un oscilloscope, un enregistreur graphique...

Lors d'une phase d'étalonnage, en réglant convenablement les gains K1 et K2, la tension somme S peut être rendue représentative du volume pulmonaire du patient et donc de la ventilation. C'est surtout cette phase d'étalonnage, à effectuer à chaque séance, qui rend l'appareil fastidieux à utiliser.

Actuellement, une personne souffrant d'une insuffisance respiratoire chronique a en fait peu de chances de guérir totalement. Son état général s'améliore tant qu'elle suit régulièrement des séances de rééducation, mais son état se dégrade à nouveau quand elle s'arrête. Il est donc nécessaire qu'une personne souffrant d'une insuffisance respiratoire chronique suive régulièrement des séances de rééducation ce qui est coûteux en temps et contraignant.

Un objet de la présente invention est de prévoir une rééducation respiratoire pouvant être effectuée en partie à domicile.

Un autre objet de la présente invention est de prévoir une rééducation respiratoire pouvant aboutir à une guérison.

Un autre objet de la présente invention est de prévoir un appareil de rééducation respiratoire.

Pour atteindre ces objets, la présente invention prévoit un procédé de rééducation respiratoire comprenant les étapes suivantes : mesurer l'amplitude respiratoire d'un patient ; acquérir un cycle de référence représentant l'amplitude en fonction du temps d'un cycle respiratoire régulier ; déterminer des cycles supérieur et inférieur encadrant le cycle de référence ; afficher en permanence sur un écran un couple de cycles supérieur et inférieur ; et afficher sur le même écran un point se déplaçant dans une direction en fonction de l'amplitude respiratoire et dans une direction orthogonale en fonction du temps.

Selon un mode de réalisation de la présente invention, le procédé comprend l'étape consistant à faire balayer l'écran horizontalement par ledit point et à réinitialiser un balayage lorsque le début d'une inspiration est détecté, la vitesse de balayage étant telle que la dimension horizontale de l'écran corresponde à une durée légèrement supérieure à la péribde du cycle de référence.

Selon un mode de réalisation de la présente invention, le procédé comprend l'étape consistant à déterminer les cycles supérieur et inférieur de manière que la distance minimale les séparant croisse progressivement dans le sens des temps croissants.

Selon un mode de réalisation de la présente invention, les maximums des cycles supérieur, inférieur et de référence sont choisis alignés selon la direction des amplitudes.

Selon un mode de réalisation de la présente invention, les cycles supérieur et inférieur sont déterminés selon les étapes suivantes : choisir un nuage de points de référence correspondant à des amplitudes du cycle de référence pour des instants distincts répartis entre un début de balayage et une fin de balayage ; déterminer des nuages de points supérieur et inférieur à partir du nuage de référence, chaque amplitude étant obtenue en additionnant et en soustrayant respectivement à l'amplitude d'un point de référence un terme formé de la somme d'une constante et d'un facteur proportionnel à la valeur absolue de la pente au point de référence ; et lisser les nuages supérieur et inférieur.

Un appareil selon la présente invention permettant la mise en oeuvre de ce procédé comprend un moyen de mesure de l'amplitude respiratoire d'un patient ; des moyens d'acquisition d'un cycle de référence représentant l'amplitude en fonction du temps d'un cycle respiratoire régulier ; des moyens de calcul et de mémorisation de cycles supérieur et inférieur encadrant le cycle de référence ; un écran sur lequel est en permanence affiché un couple de cycles supérieur et inférieur ; et des moyens de traitement permettant d'afficher sur le même écran un point se déplaçant dans une direction en fonction de l'amplitude respiratoire et dans une direction orthogonale en fonction du temps.

Selon un mode de réalisation de la présente invention, l'appareil comprend un pléthysmographe à spires inductives thoracique et abdominale relié à un microordinateur par l'intermédiaire d'une carte de conversion analogique/numérique.

Selon un mode de réalisation de la présente invention, les amplitudes respiratoires correspondent à des volumes pulmonaires.

Selon un mode de réalisation de la présente invention, les spires inductives sont intégrées dans un maillot de corps inextensible dans le sens de la hauteur et à entre-jambes.

Ces objets, caractéristiques et avantages ainsi que d'autres de la présente invention seront exposés en détail dans la description suivante de modes de réalisation particuliers faite en relation avec les figures jointes parmi lesquelles :

la figure 1, précédemment décrite, illustre un pléthysmographe à spires inductives classique qui sera utilisé dans la présente invention ;
la figure 2 illustre une courbe d'évolution de volume pulmonaire au cours d'un cycle respiratoire ;
la figure 3 représente un mode de réalisation d'appareil de rééducation selon la présente invention ; et
la figure 4 illustre un maillot de corps incorporant les spires inductives du pléthysmographe de la figure 1 permettant un emploi simple de l'appareil de la figure 3.

Le procédé selon l'invention est basé sur les deux observations suivantes.

- Des études réalisées par les inventeurs publiées dans Respiratory Physiology 75, 1989, "Individuality of breathing patterns in adults assessed over the time", ont démontré que chaque personne possède un type ventilatoire qui lui est propre et qui se conserve à long terme. Ce type ventilatoire est défini par des paramètres de fréquence, d'amplitude et de forme de cycle respiratoire.
- Il est possible d'apprendre à une personne un nouveau type ventilatoire pourvu que ce nouveau type tienne compte des caractéristiques ventilatoires propres à cette personne, notamment la forme de cycle, et pourvu que l'apprentissage s'effectue de manière suffisamment régulière pendant suffisamment longtemps.

La figure 2 illustre une courbe d'évolution typique au cours du temps du volume pulmonaire V d'une personne au repos pendant un cycle respiratoire (une inspiration suivie d'une expiration). Cette courbe peut être directement établie à partir du signal somme S fourni par le pléthysmographe de la figure 1. A un instant $t_0$ débute une phase d'inspiration I, le volume pulmonaire augmente progressivement et atteint un maximum à un instant $t_1$ à la fin de la phase d'inspiration I. A l'instant $t_1$ débute la phase d'expiration E. Le volume pulmonaire diminue progressivement, présente un point d'inflexion et rejoint approximativement la valeur initiale à un instant $t_0+T$ où recommence une nouvelle phase d'inspiration, et ainsi de suite. La phase d'expiration est généralement, comme cela est représenté, plus longue que la phase d'inspiration.

La courbe de la figure 2, considérée sans échelles d'amplitude et de temps, représente une forme de cycle respiratoire. En d'autres termes, on considère qu'une forme de cycle respiratoire est conservée lorsque la courbe subit des déformations selon des lois linéaires.

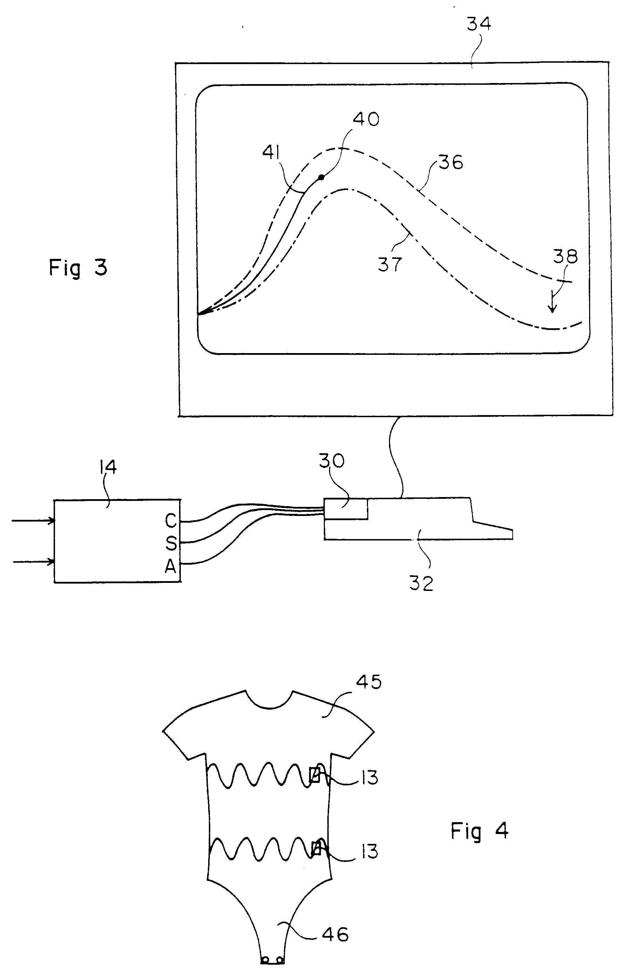

La figure 3 représente un mode de réalisation d'un appareil de rééducation respiratoire selon l'invention. On retrouve le circuit de traitement 14 du pléthysmographe de la figure 1, relié aux spires thoracique et abdominale (non représentées). Les signaux de section thoracique C, de section abdominale A et leur signal somme S sont fournis à une carte de conversion analogique/numérique 30 connectée à un microordinateur 32. Un programme adéquat exploite les signaux C, S et A convertis par la carte 30. Classiquement, ces trois signaux numérisés peuvent être mémorisés sur un intervalle de temps donné et être affichés sur un écran 34 en utilisant le microordinateur comme un oscilloscope.

Le procédé selon l'invention utilise seulement un signal représentatif du volume pulmonaire d'un patient, ce signal pouvant être le signal somme S fourni par le pléthysmographe ou calculé par le programme d'exploitation à partir des signaux thoracique C et abdominal A.

Selon la présente invention, le programme d'exploitation sera réalisé, ce qui est à la portée de tout programmeur, pour mettre en oeuvre les étapes suivantes.

- Initialement, le kinésithérapeute mesure le volume pulmonaire du patient respirant normalement au repos et plusieurs cycles respiratoires successifs sont affichés sur l'écran 34 et provisoirement mémorisés. Le kinésithérapeute choisit et mémorise, parmi les cycles affichés, un cycle de référence correspondant à une bonne ventilation, par exemple, un cycle de période et d'amplitude relativement grandes. Le cycle choisi ne doit toutefois pas être anormal, à savoir, ne pas correspondre à une ventilation excessive, par exemple un soupir. Le programme calcule à partir de ce cycle de référence des cycles de délimitation supérieur 36 et inférieur 37 encadrant le cycle de référence. Un mode d'obtention de ces cycles de délimitation est exposé ultérieurement.

- Les cycles de délimitation 36, 37 sont affichés en permanence et de manière constante sur l'écran 34, comme cela est représenté, de sorte que le début du cycle de référence commence du côté gauche de l'écran et que la fin du cycle, qui est, par exemple, indiquée par une flèche 38, se termine un peu avant le côté droit de l'écran. Ces cycles 36, 37 correspondent en fait à un gabarit de forme de cycle respiratoire.

- Le volume pulmonaire du patient est mesuré à l'aide du pléthysmographe et son évolution est affichée en temps réel sur l'écran 34 de manière que le patient voie sur l'écran 34 une tache lumineuse se déplaçant horizontalement vers la droite à une vitesse constante et verticalement en fonction du volume pulmonaire. Pour que le patient visualise mieux la forme de son cycle respiratoire, la tache 40 peut tracer derrière elle une courbe 41 correspondant aux points successifs par lesquels la tache 40 est passée depuis le début du cycle. La tache 40 se déplace vers la droite jusqu'au moment où le patient recommence une inspiration (normalement au voisinage de la flèche 38). Alors, la courbe 41 s'efface et la tache repart au début de l'écran (à gauche). Le début d'une inspiration peut être détecté par un algorithme de détection de passage par un minimum à la portée de tout programmeur.

- Le patient s'efforce lors de plusieurs séances qu'il peut effectuer à domicile de contrôler sa respiration pour que la tache 40 reste à l'intérieur du gabarit 36, 37, ce qu'il réalisera sans trop d'effort car il respirera selon une forme de cycle qui lui est spontanée.

Après une série de séances à domicile, on recommence à l'étape initiale, c'est-à-dire que le kinésithérapeute visualise une nouvelle série de cycles respiratoires du patient pour en choisir et mémoriser un qui présente de bonnes caractéristiques. En principe, le patient aura pratiquement "appris" à respirer selon la forme du cycle de référence précédemment mémorisé et les cycles mesurés correspondront en moyenne à ce cycle de référence. Généralement, un ou plusieurs cycles présenteront des meilleures caractéristiques ventilatoires. Le kinésithérapeute enregistre alors l'un de ces cycles et le patient recommencera une série de séances de rééducation à domicile avec un nouveau gabarit calculé à partir de ce cycle.

Pour que le kinésithérapeute surveille plus concrètement les progrès du patient, le programme pourra enregistrer des informations représentatives des résultats des séances sur un support magnétique tel qu'une disquette, une carte magnétique ou tout autre support d'information. Ces informations comprennent par exemple les paramètres des séances successives, tels que les dates, les durées des séances, des indications sur le taux de réussite de suivi du gabarit.

Ainsi, au cours d'une rééducation selon l'invention, le patient respirera d'une façon qui lui est adaptée puisqu'il respirera selon une forme qui a été produite par lui. Par le fait qu'il visualise ses cycles et qu'il doit quand même se contrôler pour suivre un gabarit, il prendra peu à peu conscience de la façon de contrôler ses mouvements ventilatoires. Lorsque le patient aura effectué un certain nombre de séances, il arrivera à contrôler ses mouvements ventilatoires de manière aisée et pourra de lui-même, ou même inconsciemment, les corriger et tendre vers des mouvements plus efficaces. Ces mouvements plus efficaces seront remarqués par le kinésithérapeute d'une série de séances à une autre et seront enregistrés pour former les nouveaux gabarits.

Lorsque le patient produira une forme de cycle particulièrement bonne (que l'on juge ainsi si le patient dit se sentir mieux et est capable de reproduire cette forme de façon répétitive) il pourra l'apprendre définitivement en travaillant un certain temps sur un gabarit établi à partir de ce cycle.

Cette rééducation nécessite une certaine assiduité du patient, mais celle-ci est facilitée par le fait que le patient peut effectuer une grande partie de cette rééducation à domicile.

De préférence, comme cela est représenté à la figure 3, les cycles de délimitation 36, 37 sont déterminés de manière que la distance minimale les séparant est nulle pour le début du cycle de référence et croît progressivement vers une valeur maximale à la fin du cycle de référence. La distance minimale à un instant donné est définie comme la distance entre les cycles de délimitation mesurée selon la normale au cycle de référence à cet instant.

Etant donné que les erreurs de suivi par un patient d'un gabarit de respiration sont cumulatives, un gabarit constitué des cycles de délimitation ainsi déterminés est particulièrement commode à respecter car le patient disposera à tout instant de suffisamment de marge de manoeuvre. De plus, le patient ne sera pas obligé, le cas échéant, de terminer abruptement son cycle avant la fin du cycle de référence (indiqué par la flèche), ce qui est bénéfique si l'on cherche à prolonger les cycles respiratoires du patient.

En pratique, les points des cycles de délimitation ne sont pas déterminés pour chaque point du cycle de référence. On préférera échantillonner quelques points du cycle de référence, calculer à partir de ces points les points correspondants des cycles de délimitation, et déterminer des courbes de lissage des points calculés pour obtenir chacun des cycles de délimitation. On évite ainsi de créer des cycles de délimitation qui reproduisent d'éventuels artéfacts du cycle de référence, tels que produits par une déglutition.

Pour obtenir simplement la forme susmentionnée du gabarit, on pourra calculer les n+1 premiers points des cycles de délimitation de la manière approchée suivante :
pour chaque point échantillonné $(y_i, t_i)$ où $y_i$ désigne l'amplitude à un instant d'échantillonnage $t_i$ $(0<i<n)$,

- calculer le point $(yM_i, t_i)$ correspondant du cycle supérieur selon les expressions :

$$yM_0 = y_0,$$

$$yM_i = y_i + C + k|y_i - y_{i\_1}|/(t_i - t_{i\_1}), \text{ et}$$

- calculer le point $(ym_i, t_i)$ correspondant du cycle inférieur selon les expressions :

$$ym_0 = y_0,$$

$$ym_i = y_i - C - k|y_i - y_{i\_1}|/(t_i - t_{i\_1}),$$

où C est une constante correspondant à la distance minimale entre les cycles de délimitation au niveau de l'amplitude maximale du cycle de référence, et où k désigne un coefficient fixe tel que la distance minimale entre les cycles de délimitation croisse progressivement en fonction des temps $t_i$. Les derniers points des cycles de délimitation pourront être choisis arbitrairement pour obtenir un évasement convenable du gabarit à la fin du cycle de référence.

Le pléthysmographe à spires tel que décrit à la figure 1 ne convient pas tout à fait à une utilisation à domicile. En effet, la précision des mesures peut varier d'une séance à une autre en fonction du positionnement des spires thoracique 10 et abdominale 11 par rapport à une séance précédente. Ainsi, le pléthysmographe devrait être étalonné à chaque séance (réglage des gains K1 et K2 des convertisseurs fréquence-tension 15 et 16) afin que l'on puisse s'attendre à des résultats comparables d'une séance à une autre.

La figure 4 illustre une solution permettant une bonne répétitivité du positionnement des spires du pléthysmographe d'une séance à une autre afin que les réglages soient effectués une fois pour toutes à la première séance. Les spires sont intégrées dans le tissu d'un maillot de corps 45. Le tissu de ce maillot de corps est inextensible dans le sens de la hauteur et un entre-jambes 46 empêche tout déplacement vertical du maillot de corps. Avec une telle disposition, on est sûr que les spires retrouvent pratiquement les mêmes positions d'une séance à l'autre. Les oscillateurs 13 seront munis de connecteurs afin de pouvoir les détacher du maillot de corps pour laver ce dernier. L'entre-jambes 46 sera réglable pour une meilleure adaptation à l'anatomie du patient.

Dans une autre version de l'appareil de rééducation selon la présente invention, les fonctions réalisées par le microordinateur 32 et la carte de conversion 30 pourront être réalisés par un microcontrôleur spécifique intégré dans un appareil de dimensions plus petites qu'un microordinateur et que l'on pourra brancher sur un téléviseur.

De nombreuses variantes et modifications de la présente invention apparaîtront à l'homme de l'art. On pourra notamment réaliser un appareil de rééducation plus rudimentaire comprenant par exemple un oscilloscope sur l'écran duquel on applique une feuille transparente portant le dessin du gabarit.

**Revendications**

1. Procédé de rééducation respiratoire comprenant l'étape consistant à mesurer l'amplitude respiratoire d'un patient ;
   caractérisé en ce qu'il comprend les étapes suivantes :

   - acquérir un cycle de référence représentant l'amplitude en fonction du temps d'un cycle respiratoire régulier ;
   - déterminer des cycles supérieur (36) et inférieur (37) encadrant le cycle de référence ;
   - afficher en permanence sur un écran (34) un couple de cycles supérieur (36) et inférieur (37) ; et
   - afficher sur l même écran un point (40) se déplaçant dans une direction en fonction de l'amplitude respiratoire et dans une direction orthogonale en fonction du temps.

2. Procédé selon la revendication 1, caractérisé en ce qu'il comprend l'étape consistant à faire balayer l'écran (34) horizontalement par ledit point (40) et à réinitialiser un balayage lorsque le début d'une inspiration est détecté, la vitesse de balayage étant telle que la dimension horizontale de l'écran corresponde à une durée légèrement supérieure à la période du cycle de référence.

3. Procédé selon la revendication 1, caractérisé en ce qu'il comprend l'étape consistant à déterminer les cycles supérieur (36) et inférieur (37) de manière que la distance minimale les séparant croisse progressivement dans le sens des temps croissants.

4. Procédé selon la revendication 3 caractérisé en ce que les maximums des cycles supérieur (36), inférieur (37) et de référence sont alignés selon la direction des amplitudes.

5. Procédé selon la revendication 4, caractérisé en ce que les cycles supérieur (36) et inférieur (37) sont déterminés selon les étapes suivantes :

   - choisir un nuage de points de référence correspondant à des amplitudes $(y_i)$ du cycle de référence pour des instants distincts $(t_i)$ répartis

entre un début de balayage et une fin de balayage ;
- déterminer des nuages de points supérieur et inférieur à partir du nuage de référence, chaque amplitude ($yM_i$, $ym_i$) étant obtenue en additionnant et en soustrayant respectivement à l'amplitude d'un point de référence un terme formé de la somme d'une constante et d'un facteur proportionnel à la valeur absolue de la pente au point de référence ; et
- lisser les nuages supérieur et inférieur.

6. Procédé selon la revendication 1, caractérisé en ce que les amplitudes respiratoires correspondent à des volumes pulmonaires.

7. Dispositif de rééducation respiratoire comprenant un moyen de mesure (10, 11, 13, 14) de l'amplitude respiratoire d'un patient ;
caractérisé en ce qu'il comprend :

- des moyens d'acquisition (30, 32) d'un cycle de référence représentant l'amplitude en fonction du temps d'un cycle respiratoire régulier ;
- des moyens (32) de calcul et de mémorisation de cycles supérieur (36) et inférieur (37) encadrant le cycle de référence ;
- un écran (34) sur lequel est en permanence affiché un couple de cycles supérieur et inférieur ; et
- des moyens de traitement (32) permettant d'afficher sur le même écran un point (40) se déplaçant dans une direction en fonction de l'amplitude respiratoire et dans une direction orthogonale en fonction du temps.

8. Dispositif selon la revendication 7, caractérisé en ce qu'il comprend un pléthysmographe à spires inductives thoracique (10) et abdominale (11) relié à un microordinateur (32) par l'intermédiaire d'une carte de conversion analogique/numérique (30).

9. Dispositif selon la revendication 7, caractérisé en ce que les amplitudes respiratoires correspondent à des volumes pulmonaires.

10. Dispositif selon la revendication 7, caractérisé en ce que les spires inductives (10, 11) sont intégrées dans un maillot de corps (45) inextensible dans le sens de la hauteur et à entre-jambes (46).

**Claims**

1. A breathing rehabilitation method including the step of measuring the breathing amplitude of a patient; characterized in that it includes the following steps:

- acquiring a reference cycle representative of the amplitude as a function of time of a regular breathing cycle;
- determining upper (36) and lower (37) cycles enclosing the reference cycle;
- permanently displaying on a screen (34) a pair of upper (36) and lower cycles (37); and
- displaying on the same screen a spot (40) moving in one direction as a function of the breathing amplitude and in a perpendicular direction as a function of time.

2. The method of claim 1, characterized in that it includes the step of horizontally scanning the screen (34) with said spot (40) and resetting the scanning when the beginning of an inhalation is detected, the scanning speed being such that the horizontal length of the screen corresponds to a period slightly greater than the period of the reference cycle.

3. The method of claim 1, characterized in that it includes the step of determining the upper (36) and lower (37) cycles so that the minimum distance separating them progressively increases in the direction of increasing time.

4. The method of claim 3, characterized in that the maximum values of the upper (36), lower (37) and reference cycles are aligned in the amplitude direction.

5. The method of claim 4, characterized in that said upper (36) and lower (37) cycles are determined according to the following steps:

- choosing a cloud of reference points corresponding to amplitudes ($y_i$) of the reference cycle for distinct times ($t_i$) distributed between the beginning and the end of a scan;
- determining clouds of upper and lower points from the reference cloud, each amplitude ($yM_i$, $ym_i$) being obtained by adding and subtracting respectively from the amplitude of a reference point a term constituted by the sum of a constant and of a factor that is proportional to the absolute value of the slope at the reference point; and
- smoothing the upper and lower clouds.

6. The method of claim 1, characterized in that the breathing amplitude corresponds to a pulmonary volume.

7. A device for breathing rehabilitation, including means (10, 11, 13, 14) for measuring the breathing amplitude of a patient; characterized in that it includes:

- means (30, 32) for acquiring a reference cycle representing the amplitude as a function of time of a regular breathing cycle;
- means (32) for calculating and storing upper (36) and lower (37) cycles enclosing the reference cycle;
- a screen (34) on which a pair of upper and lower cycles is permanently displayed; and
- processing means (32) for displaying on the same screen a spot (40) moving in one direction as a function of the breathing amplitude and in a perpendicular direction as a function of time.

8. The device of claim 7, characterized in that it includes a plethysmograph that has thoracic (10) and abdominal (11) inductive windings and is connected to a microcomputer (32) through an analog/digital conversion board (30).

9. The device of claim 7, characterized in that the breathing amplitudes correspond to pulmonary volumes.

10. The device of claim 7, characterized in that the inductive windings (10, 11) are integrated in a vertically non-stretch vest (45) including a pelvic flap (46).

**Patentansprüche**

1. Verfahren zur Atmungsumschulung, beinhaltend den Schritt Messen der Atmungsamplitude eines Patienten;
dadurch gekennzeichnet, daß es die folgenden Schritte umfaßt:

- Erfassen eines Referenzzyklus, der die Amplitude als Funktion der Zeit eines regulären Atmungszyklus darstellt;
- Bestimmen eines oberen (36) und eines unteren (37) Zyklus, die den Referenzzyklus einschließen;
- dauerhaftes Anzeigen eines Paares des oberen (36) und unteren (37) Zyklus auf einem Bildschirm (34); und
- Anzeigen eines Punktes (40), der sich in einer Richtung als Funktion der Atmungsamplitude und in einer orthogonalen Richtung als Funktion der Zeit bewegt, auf demselben Bildschirm.

2. Verfahren nach Anspruchs 1, gekennzeichnet durch den Schritt horizontales Abtasten des Bildschirms (34) durch den Punkt (40) und Reinitialisieren einer Abtastung, wenn der Beginn einer Einatmung erfaßt wird, wobei die Geschwindigkeit der Abtastung so ist, daß die horizontale Länge des Bildschirms einer Zeitdauer entspricht, die geringfügig größer als eine Periode des Referenzzyklus ist.

3. Verfahren nach Anspruch 1, gekennzeichnet durch den Schritt Bestimmen des oberen (36) und unteren (37) Zyklus in der Weise, daß ihr gegenseitiger Minimalabstand mit zunehmender Zeit fortschreitend zunimmt.

4. Verfahren nach Anspruch 3, dadurch gekennzeichnet, daß die Maxima des oberen Zyklus (36), des unteren Zyklus (37) und des Referenzzyklus in Richtung der Amplituden ausgerichtet sind.

5. Verfahren nach Anspruch 4, dadurch gekennzeichnet, daß der obere (36) und der untere (37) Zyklus gemäß den folgenden Schritten bestimmt werden:

- Wählen einer Gruppe von Referenzpunkten, die Amplituden $(y_i)$ des Referenzzyklus für unterschiedliche Zeitpunkte $(t_i)$, welche zwischen einem Abtastungsbeginn und einem Abtastungsende verteilt sind, entsprechen;
- Bestimmen von Gruppen von oberen und unteren Punkten in bezug auf die Referenzgruppe, wobei jede Amplitude $(yM_i, ym_i)$ erhalten wird durch Addieren und Subtrahieren eines Gliedes, das aus der Summe einer Konstante und eines Faktors gebildet wird, welcher zu dem Absolutwert der Steigung in dem Referenzpunkt proportional ist, zu bzw. von der Amplitude eines Referenzpunktes; und
- Glätten der oberen und der unteren Gruppe.

6. Verfahren nach Anspruch 1, dadurch gekennzeichnet, daß die Atmungsamplituden pulmonalen Volumina entsprechen.

7. Vorrichtung zur Atmungsumschulung, mit einer Einrichtung zum Messen (10, 11, 13, 14) der Atmungsamplitude eines Patienten;
dadurch gekennzeichnet, daß sie umfaßt:

- eine Einrichtung zum Erfassen (30, 32) eines Referenzzyklus, der die Amplitude als Funktion der Zeit eines regulären Atmungszyklus darstellt;
- eine Einrichtung (32) zum Berechnen und Speichern eines oberen (36) und unteren (37) Zyklus, die den Referenzzyklus einschließen;
- einen Bildschirm (34), auf dem ein Paar des oberen und unteren Zyklus permanent angezeigt wird, und
- eine Verarbeitungseinrichtung (32), die gestattet, einen Punkt (40), der sich in einer Richtung als Funktion der Atmungsamplitude und in einer orthogonalen Richtung als Funktion der Zeit bewegt, auf demselben Bildschirm anzuzeigen.

8. Vorrichtung nach Anspruch 7, dadurch gekennzeichnet, daß sie einen Plethysmograph mit thorakalen (10) und abdominalen (11) induktiven Windungen enthält, die mit einem Mikrocomputer (32) über eine Analog/Digital-Wandlerkarte (30) verbunden sind.

9. Vorrichtung nach Anspruch 7, dadurch gekennzeichnet, daß die Atmungsamplituden pulmonalen Volumina entsprechen.

10. Verfahren nach Anspruch 7, dadurch gekennzeichnet, daß die induktiven Windungen (10, 11) in ein in Höhenrichtung nicht dehnbares Leibtrikot (45) mit einer Schrittlasche (46) integriert sind.

Fig. 1

Fig. 2

Fig 3

34

40

41

36

37

38

14

C
S
A

30

32

45

13

13

Fig 4

46